# EUROPEAN PATENT APPLICATION

(11) **EP 4 014 879 A1**
(43) Date of publication of application: **22.06.2022**
(21) Application number: 20214451.5
(22) Date of filing: 16.12.2020
(51) Int. Cl.: A61B 6/08, A61B 6/00

(54) **FIELD OF VIEW VISUALIZATION FOR PHASE CONTRAST X-RAY IMAGING SYSTEMS**

(71) Applicant: Koninklijke Philips N.V., 5656 AG Eindhoven (NL)
(72) Inventor: KOEHLER, Thomas, 5656 AE Eindhoven (NL); LOESCHER, Stefan Leonhard, 5656 AE Eindhoven (NL); VOGTMEIER, Gereon, 5656 AE Eindhoven (NL); YAROSHENKO, Andriy, 5656 AE Eindhoven (NL)
(74) Representative: Philips Intellectual Property & Standards

(57) **Abstract**

A phase contrast X-ray imaging system with field of view (FOV) visualization and a holder arranged to hold a grating and a light field projector to enable the FOV illumination, for an operator to visualize the region on a subject that will be irradiated with X-rays. By taking advantage of the holder of the necessary object in the X-ray beam path (i.e. the grating) to hold a light field projector to illuminate the FOV, the solution is naturally more compact to implement and the mechanical complexity is kept to a minimum. The light field projector provides a sharp, well-defined area with a distinct outline of the FOV.

## Description

### FIELD OF THE INVENTION

The invention relates to the field of phase contrast X-ray imaging which includes phase contrast and dark-field X-ray imaging. The invention relates to a phase contrast X-ray imaging system with field of view (FOV) visualization and a holder to allow the integration of the FOV visualization functionality, such that an area to be irradiated with X-rays on a subject is visualized.

### BACKGROUND OF THE INVENTION

Phase contrast X-ray imaging (PCI) offers greater sensitivity with respect to conventional X-ray imaging, enabling the imaging of low-density materials non-destructively. These materials are present across a broad range of applications, included but not limited to security, building and construction, electronics and medical applications. For the latter, phase contrast X-ray imaging systems can potentially offer an advantage in clinical applications such as mammography, lung imaging, and bone imaging, because of its capability of imaging subtle differences in material composition which is otherwise unavailable or only available in lower quality using traditional x-ray imaging techniques.

There exist several PCI techniques among which the Talbot interferometry (grating-based imaging) and edge illumination (EI, sometimes referred to as coded aperture technique) are particularly attractive due to the possibility to implement them with conventional X-ray sources as those required for application in commercial systems.

Grating-based phase contrast X-ray imaging (PCI) requires one or more grating components. Exemplarily, an interference pattern created by a first or phase grating component can be analyzed using a second or analyzer grating component which is moved at various positions from which intensity variations are recorded at a detector. An optional source grating component may be included to enable the use of low coherence X-ray sources, for instance such as those normally used in medical imaging. By placing an object in the interferometer arrangement (e.g. between source grating and phase grating or between phase grating and analyzer grating) along the X-ray beam path, a waveform as a function of the analyzer grating position can be recorded at the detector and compared to the reference case without object in the X-ray beam path. The resulting waveforms will differ in several aspects, namely in mean intensity, phase and amplitude from which complementary information relating to attenuation, phase, and dark-field can be obtained.

The EI approach is based on the fact that direction of X-rays when interacting with an object in the X-ray beam path experience small deviations caused by phase shifts. By illuminating only an edge of a detector pixel the effect is enhanced. For example, photons that would have hit a detector pixel surface in the absence of the object, experience small deviations in the presence of the object that are enough to deflect them outside such pixel surface. To implement and being able to exploit this effect, typically a pair of multiple edge objects acting as coded apertures, or in other words, absorbing gratings, are used. In the coded aperture systems literature these objects are often referred to as coded-aperture masks; in the context of the presently claimed invention this term will be also understood as an absorbing grating and the terms will be used interchangeably. One of the coded-aperture masks or absorbing gratings is placed between the X-ray source and the object (so-called sample mask, or a sample absorbing grating), to create a plurality of individual X-ray beams, each one hitting one of the pixel edges created by the second, so-called detector mask. With part of the detector pixels, i.e. the pixel edges, masked by the detector mask, sensitive and insensitive (i.e. absorbing) to X-rays regions are defined at the detector. By measuring and comparing an illumination curve with and without an object in place, extraction of attenuation, phase, and dark-field signals are possible.

Since a single, phase contrast X-ray imaging system, regardless of its implementation, offers both phase contrast (PC) and dark-field (DAX) information (in addition to attenuation/transmission X-ray information), in the context of the presently claimed invention the term phase contrast X-ray imaging should be understood as covering both PC and DAX imaging.

Because of the nature of the radiation involved in these systems, it is a requirement for an operator to collimate the X-ray beam to a desired field of view (FOV) and hence prevent undesired radiation exposure. Particularly, in medical applications, this is necessary to minimize the X-ray dose to the patient. Consequently, it is desired, and it is even a regulatory requirement in medical applications, that prior to the radiation exposure, the system is able to illuminate the region that will be irradiated with X-rays and hence make this visible to the operator for control as needed (e.g. to radiate a specific organ of a patient).

In conventional X-ray systems, the region to be radiated is indicated by a visible light source that is integrated into the collimator box that illuminates the same beam as the X-ray source. In this concept, by using a mirror in the X-ray beam path which is transparent to the wavelength of the radiation beam, the visible light, located in the vicinity of the X-ray source, is reflected such that it follows the same beam path and collimation as the X-rays. The traditional concept cannot be directly adopted in PCI systems because of the presence of the optically opaque objects in the X-ray beam path, i.e. the gratings. Either a sample absorbing grating in the case of the EI based PCI systems, or a source grating and/or a phase grating in the case of grating-based PCI systems, since at least, the presence of the phase grating is necessary. Moreover, in grating-based imaging, the so-called inverse geometry (where the subject is between phase grating and analyzer grating) may be desired because it gives the advantage that the largest grating (i.e. analyzer grating) has the least demanding specification with respect to the grating period. In this scenario, applying the concept would lead to a very bulky solution as the mirror and collimator cannot be placed in the beam path following the X-ray source because of the opaque grating(s), and therefore a farther apart positioning will be needed. The farther the light source and mirror need to be placed, the larger the mirror that will be needed for the distance to the focal spot. Hence, it is evident that the problem of visualizing the region that will be irradiated with X-rays, prior to the radiation exposure, requires special attention in PCI, where there are optically opaque gratings in the X-ray beam path.

WO2019076939 claims a system and method to address the aforementioned problem by employing two light fields that are projected towards the subject in two different light field directions and that partially overlap each other on the subject forming an overlapping light field area indicating the area to be radiated. In this invention, the light fields are decoupled from the X-ray beam path and are projected to the subject each from a different path, e.g. from the sides of the radiation path, and hence, they have their own path and collimation. By avoiding the radiation path, the solution tends to occupy more space than ideally desired and can be mechanically complex. Moreover, the accuracy of the solution to indicate the area to be radiated can be further improved. A simpler, more compact solution while keeping the mechanical complexity to a minimum, and which in addition can offer an increased accuracy is desired.

### SUMMARY OF THE INVENTION

An even more advanced solution is needed to solve the illumination problem in the presence of opaque gratings. This is achieved by the subject matter of each of the independent claims. Further embodiments of the invention are described in the respective dependent claims.

Embodiments according to the present invention are directed to a phase contrast X-ray imaging system with field of view (FOV) visualization. The system comprises an X-ray source configured to emit an X-ray beam through an examination region towards a target area located within the examination region, an X-ray detector position on an opposite side of the examination region from the X-ray source, an X-ray beam shaper configured to shape the X-ray beam to irradiate a radiation area on the target area, a grating positioned between the X-ray source and the detector, a light field projector configured to project a light field in the visible spectrum on the target area, where the projected light field corresponds to the radiation area and a holder arranged to hold the light field projector and the grating. Instead of avoiding the radiation path, by taking advantage of the holder of the necessary object in the X-ray beam path (i.e. the grating) to hold a light field projector to illuminate the FOV, the solution is naturally more compact to implement as practically no additional housing is needed and the mechanical complexity is kept to a minimum.

In an embodiment the holder contains a first part arranged to hold the light field projector and a second part arranged to hold the grating. The first part and the second part are permanently or reversibly fixed to each other. This way, a more flexible usage and deployment of the holder are allowed, where the supply and/or replacement of parts may be more convenient.

In an embodiment the grating is a phase grating or a source grating or a sample absorbing grating. In this manner, diverse PCI systems and configurations of such systems benefit from the advantages of the claimed invention. For example, a grating-based imaging system in the so-called direct geometry configuration (subject located between source grating and phase grating), a grating-based imaging system in the so-called inverse geometry configuration (subject located between phase grating and analyzer grating), or a coded aperture system.

In an embodiment a plurality of light field projectors is mounted to the holder configured to project a light field on a target area and the combined projected light fields mark an area corresponding to the radiation area. By using a plurality of light field projectors, the combination of the independently projected light fields may lead to an even more accurate FOV visualization.

In an embodiment the PCI system further comprises a distance sensor to measure a distance from a plane where the light field is projected to a plane where the target area is located. A more advanced system comprising a distance sensor to measure such distance facilitates that the light field projector may have a source point different from that of the X-ray source. Under the latter circumstances, such distance measurement enabled by the distance sensor contributes to a correct FOV visualization.

In an embodiment the light field projector is a laser device or a projector. A laser device or a projector create a sharp and well-defined area such that the FOV can be visualized with a clean and distinct outline. Further, the use of a laser device may bring an increased functionality to the system. The beam may be used when detected with additional sensors at the detector side for alignment and automatic position checks. The use of a projector may also bring an increased functionality to the system by allowing the projection of additional information such as alignment to the patient's body with projection of the body shape; based on complementary subject data, the projection of a target region (e.g. a body part) to favor the application of the correct FOV setting; or any other relevant data for the confirmation of the examination (e.g. a patient's name, purpose of scan, date of birth, etc.). Moreover, the projector could be complemented with a feedback device (e.g. a camera) that allows an operator to control the FOV setting via gesture control at the back of the subject (e.g. like action on a touch screen). Besides the increased functionality by projection of additional information, such a user interface improves the workflow and allows for a faster and more precise FOV setting which could be fully automated.

In an embodiment the light field projector is a laser device configured to project a line shape and the system further comprises a guide rail mounted to the holder, wherein said guide rail is configured to allow a movement of the laser device along the guide rail which comprises a curvature with a center that coincides with a center of a focal spot of the X-ray source. By letting a laser device moving along a guide rail sharing the same source point with respect to the X-ray source (i.e. the center of the focal spot of the X-ray source), a very accurate and relatively simple to implement indication of the borders of the FOV can be achieved through a combination of projected line shapes as long as the laser devices are in a position of the guide rail where the projected line shapes match the edges of the FOV setting of the system, e.g. the edges of collimator blades of a collimator.

In an embodiment the light field projector is configured to update the projected light field when the radiation area is changed to an updated radiation area such that the updated projected light field corresponds to the updated radiation area. This way a seamless operation of the system, favoring the workflow, is achieved.

Further embodiments of the present invention are directed towards a holder arranged to hold a grating of a phase contrast X-ray imaging system and a light field projector for projecting a light field towards a light field target area. Such a holder, having the double purpose of holding a grating and a light field projector is a simple, practical and compact way to allow a more efficient integration of FOV visualization functionality when installed in a phase contrast X-ray imaging system.

In an embodiment the holder contains a first part arranged to hold the light field projector and a second part to hold the grating, wherein the first part and the second part are permanently or reversibly fixed to each other.

In an embodiment the light field projector is a laser device or a projector.

In an embodiment the holder further comprises a guide rail mounted to it, configured to allow a movement of the light field projector along the guide rail which comprises a curvature.

In an embodiment the light field projector comprises a plurality of laser devices configured to project a line shape and are mounted to the first part of the holder through the guide rail with a spindle with threads. The threads have inverse slopes to allow, when installed in a phase contrast X-ray imaging system, a symmetric movement of the laser line shapes as projected by the laser devices with respect to an axis coincident with an optical axis of the phase contrast X-ray imaging system. Thanks to such symmetric movement of the laser devices, the position of the plurality of laser devices can be controlled in conjunction and the position of the laser devices along the guide rail can be translated directly into a required number of turns of the spindle.

In an embodiment the holder further comprises a distance sensor to measure, when installed in a phase contrast X-ray imaging system, a distance between the light field projector and the light field target area.

In an embodiment the light field projector comprises at least one laser device mounted to the first part of the holder, configured to project a collimated laser spot towards the light field target area and to scan over the light field target area such that a border of the light field target area is visualized. This embodiment constitutes an alternative to project a light field towards the light field target area without relying on the mechanical motion of the laser device, and to outline an area not necessarily based on line-shapes, but instead, through a collimated laser spot projection thanks to the laser spot scanning over the light field target area.

In an embodiment the light field projector comprises at least one projector mounted to the first part of the holder, configured to project a light field towards the light field target area to form a light field covering and delimiting the complete light field target area. In general, the use of a projector has advantages that have been mentioned earlier. A plurality of projectors may bring an even more accurate implementation because relies on a combination of independently projected light fields covering and delimiting an area whose borders lie in the regions with less parallax.

In an embodiment at least one of the at least one projector projects further information on or near the light field target area, such as projection of a target region, alignment to a subject's body with projection of a body shape, and/or other data, bringing altogether workflow improvements.
These and other aspects of the present invention will become apparent from and elucidated with reference to the embodiments described hereinafter.

### BRIEF DESCRIPTION OF THE FIGURES

In the following drawings:

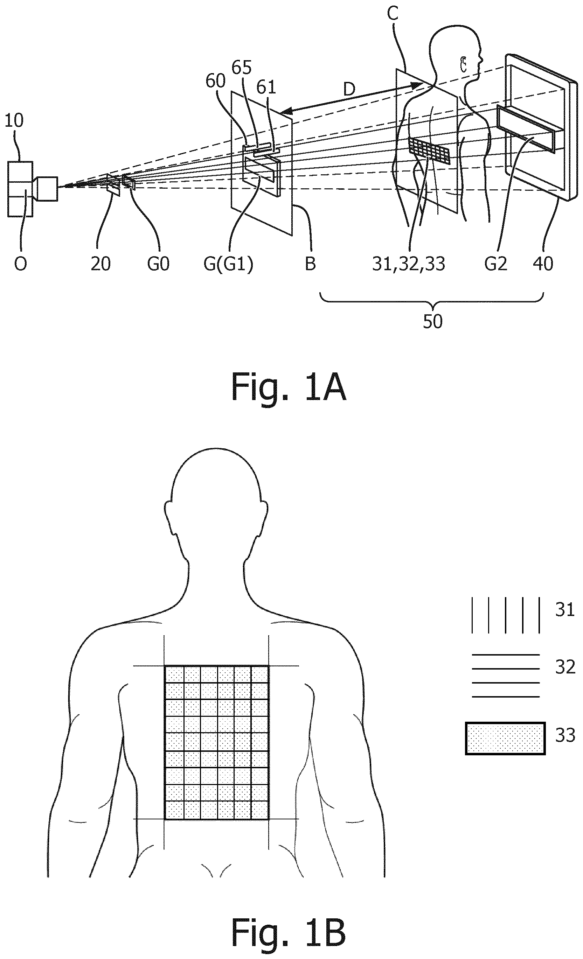

Fig. 1 shows a schematic diagram of a PCI system with FOV illumination wherein the holder of a phase grating is also arranged to hold a light field projector, as described in conjunction with the presently claimed invention. Fig. 1A shows the PCI system, Fig. 1B sketches a more detailed view of the FOV visualization of the PCI system of Fig 1A.

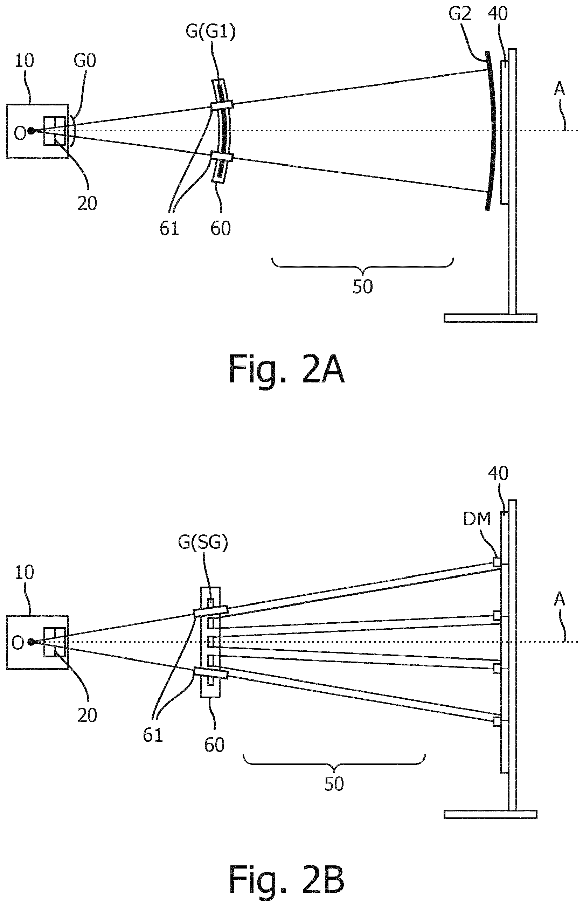

Fig. 2 shows schematic diagrams of PCI systems with FOV illumination as described in conjunction with the presently claimed invention. In Fig. 2A, the PCI system is a grating-based imaging system wherein the holder of a phase grating is also arranged to hold a light field projector. In Fig. 2B, the PCI system is an EI-based imaging system wherein the holder of the sample absorbing grating is also arranged to hold a light field projector.

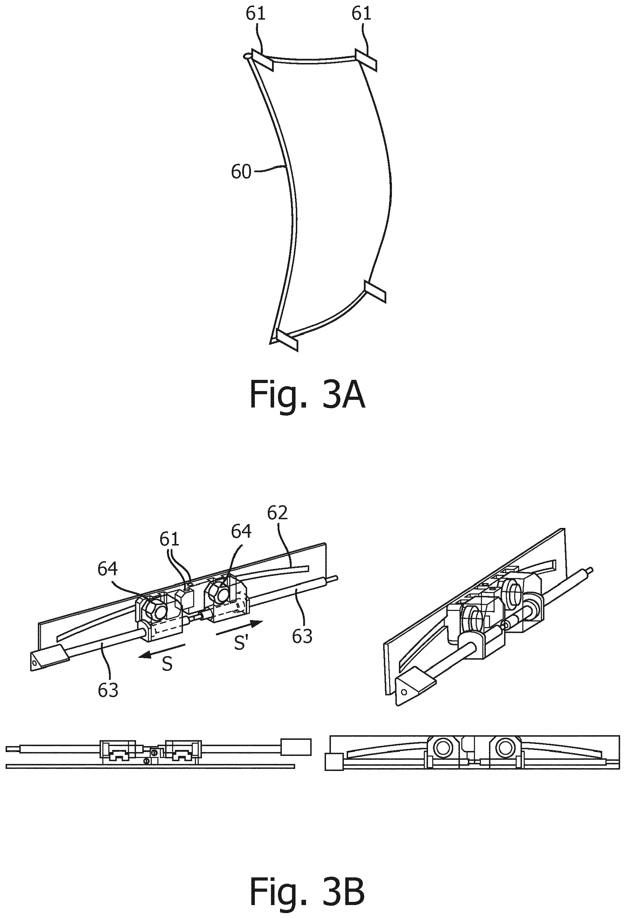

Fig. 3 illustrates aspects related to a holder as described in conjunction with the presently claimed invention. Fig. 3A shows a schematic diagram of an exemplary holder arranged to hold a grating of a PCI system and a light field projector. Fig. 3B illustrates views from different perspectives of a design concept of a guide rail with a spindle with threads to which a light field projector is mounted.

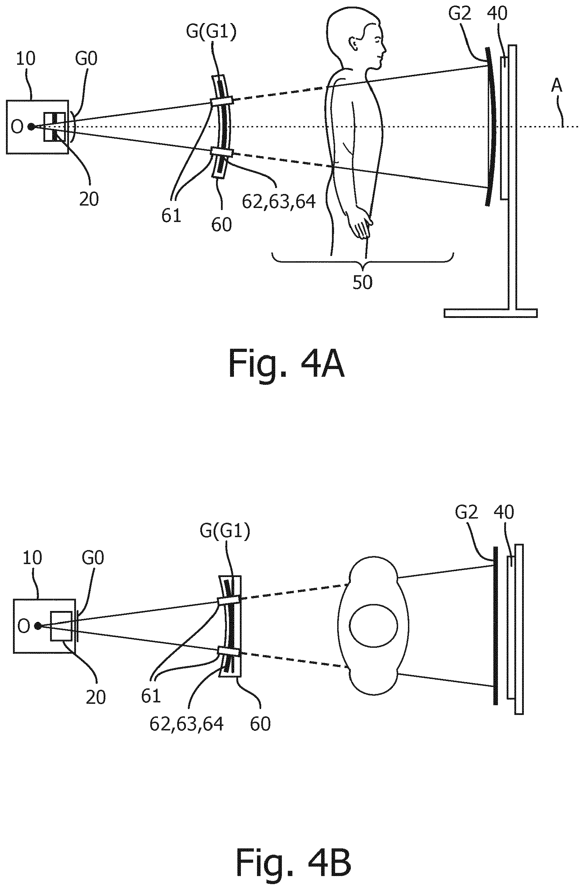

Fig. 4 shows a schematic diagram of a PCI system with FOV illumination enabled by a plurality of laser devices wherein the holder of a phase grating is also arranged to hold the light field projector, as described in conjunction with the presently claimed invention. Fig 4A is a side view and Fig. 4B is a top view of the PCI system.

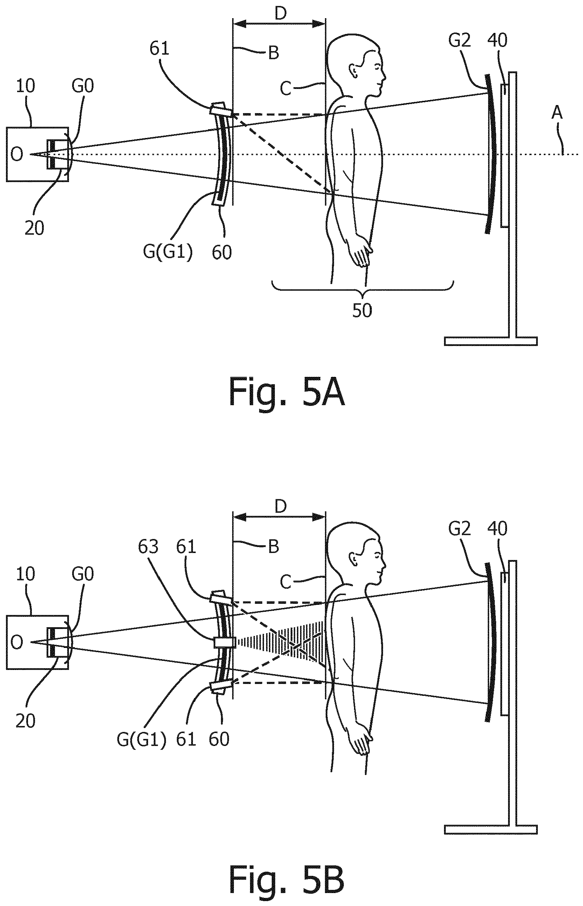

Fig. 5 shows side views of a schematic diagram of a PCI system with FOV illumination wherein the holder of phase grating G1 is also arranged to hold the light field projector, as described in conjunction with the presently claimed invention. In Fig. 5A the FOV illumination is enabled by a projector and in Fig. 5B by a plurality of projectors.

The invention may take form in various components and arrangements of components, and in various processes operations and arrangements of process operations. The drawings are only for the purpose of illustrating the preferred embodiments and are not to be construed as limiting the invention. To better visualize certain features may be omitted or dimensions may be not according to scale. Like of similar components are given the same reference numerals in different figures.

### DETAILED DESCRIPTION OF THE INVENTION

In this detailed description, various specific details are presented. Embodiments of the present invention can be carried out without these specific details. Furthermore, well-known features, elements and/or steps are not necessarily described in detail for the sake of clarity and conciseness of the present disclosure.

The presently claimed invention provides a solution to illuminate the field of view (FOV) of a phase contrast X-ray imaging (PCI) system for an operator to visualize the region on a subject that will be irradiated with X-rays, and hence be able to control the FOV as needed. It would be advantageous to offer an accurate, compact and relatively simple to implement solution for PCI systems, which have optically opaque objects in the X-ray beam path (e.g. gratings). To better address one or more of these challenges, the invention takes advantage of an already present object in the X-ray beam path to enable the FOV visualization. Therefore, in a first aspect, the invention relates to a phase contrast X-ray imaging system with FOV visualization, and in a second aspect, it relates to a holder arranged to hold the existing object in the X-ray beam path and a light field projector to enable the FOV visualization.

With reference to Fig. 1A there is shown a schematic diagram of a PCI system with FOV illumination. The PCI system comprises an X-ray source 10, with an X-ray tube focal spot center O, an X-ray beam shaper 20, and an X-ray detector 40. The X-ray source 10 is configured to emit an X-ray beam through an examination region 50 towards a target area 31 e.g. part of a patient, that is located within the examination region 50. The X-ray beam shaper 20 is configured to shape the X-ray beam to irradiate a radiation area 32 on the target area 31. The X-ray detector 40 is positioned on an opposite side of the examination region 50 from the X-ray source 10. The PCI system also comprises a grating G, for instance a phase grating G1, positioned between the X-ray source 10 and the detector 40. A holder 60 arranged to hold the grating G as well as a light field projector 61, comprise also the system. The light field projector 61 projects an outline/area of the FOV onto the subject to be imaged. To this end, the light field projector 61 is configured to project a light field in the visible spectrum on the target area 31, where the projected light field 33 corresponds to the radiation area 32. This is schematically shown in Fig. 1A and a more detailed view of the FOV visualization is sketched in Fig. 1B. The correspondence between the projected light field 33 and the radiation area 32 means that an area defined by the projected light field 33 is equivalent or nearly equivalent to the radiation area 32. In the context of this invention, the term 'equivalent' means that there is a perfect match between the area defined by the projected light field 33 and the radiation area 32. In the context of this invention, the term 'nearly equivalent' means that the match between the areas is substantial within reasonable error margins (e.g. within margins given by the regulatory authorities, or in numbers errors in the order of less or equal to 10%, preferably less or equal to 5%, more preferably less or equal to 1%), such errors being for example, a slight (i.e. less or equal to 1, 5 or 10%) difference between the areas' surfaces and/or with a slight (i.e. less or equal to 1, 5 or 10%) mismatch between the geometrical center of the areas.

In an example, the PCI system may be a grating-based imaging system as illustrated in Fig. 2A, where the so-called inverse geometry is shown. In this case, during an imaging procedure, the subject is located within the examination region 50, between phase grating G1 and analyzer grating G2. Considering this configuration, in an embodiment, and as shown in the figure, the holder 60 of phase grating G1 is also arranged to hold a light field projector 61. In another embodiment (not shown), where the so-called direct geometry is used, that is, where during an imaging procedure, the subject is located within the examination region 50, between source grating G0 and phase grating G1, the holder 60 of source grating G0 is also arranged to hold a light field projector 61.

In an example, the PCI system may be an EI-based system, i.e. a coded aperture system as illustrated in Fig. 2B. The beam created by the X-ray source 10 is subdivided in a plurality of individual beams by the sample absorbing grating SG (the so-called sample mask). Thereby the absorbing grating SG can act both as a beam shaper 20 and as a grating G, potentially simplifying the system by making optional the need for a dedicated beam shaper 20. In an embodiment, and as shown in the figure, the holder 60 of the sample absorbing grating SG is also arranged to hold a light field projector 61. In this case, during an imaging procedure, the subject is located between the sample absorbing grating SG and the detector mask DM.

From the examples aforementioned, in analogy, it is clear that alternative implementations of PCI systems involving the use of optically opaque objects in the X-ray beam path (such as gratings G), will benefit from the currently claimed invention. Since the spirit of the presently claimed invention relies on a holder 60 of an object G that needs to be located in the X-ray beam path, then, a holder 60 of an X-ray beam shaper 20 (e.g. a collimator) of a conventional X-ray imaging system can be also arranged to hold a light field projector 61 and hence such systems can also benefit from the advantages of the currently claimed invention. By taking advantage of the holder 60 of the necessary object in the X-ray beam path (for instance the holder 60 of the grating G), the solution is naturally more compact to implement as practically no additional housing is needed.

In the context of the claimed invention, the term light field projector refers to a device for projecting a light field in the visible spectrum. The light field projector 61 has the purpose of visualizing the FOV as schematically illustrated in Fig. 1B. In the figure, a pattern of vertical lines depicts the target area 31, e.g. part of a patient, and a pattern of horizontal lines depicts the radiation area 32 to be irradiated with X-rays, according to the FOV of the PCI system. The light field projector 61 projects a light field towards a light field target area 33 which corresponds to the radiation area 32, thereby enabling the visualization of the FOV. As illustrated in Fig. 1B, the visualization of the FOV 33 may be realized by covering and delimiting (dotted region), but it may be also realized by its projected borders (borders of dotted region).

Examples of light field projectors 61 include but are not limited to a projector or a laser device. There exist a variety of projectors 61, these may depend on the source of light they use (e.g. lamp, LED or laser) and the technology for projection; some examples include DLP (Digital Light Processing), LCD (Liquid Crystal Displays), LED, LCoS (Liquid Crystal on Silicon), or laser projectors. Further, they may be full-size projectors, but they may be also handheld projectors (e.g. mini projector, pico projector); the smaller the better may be the preferred for the currently claimed invention as they will take up limited space and are lightweight thereby being more convenient for the supporting equipment, such as the holder 60. A laser device is defined as a device comprising one or more lasers as a source of light, and means for laser beam shaping (e.g. optical lenses, mirrors) into any arbitrary shape or pattern (e.g. a line, a point, a cross, an edge, a rectangle, etc.). Other types of light field projectors 61 may be considered especially if they can create a sharp and well-defined area.

Turning now to the holder 60, a schematic diagram of an exemplary holder 60 is shown in Fig. 3A. The holder 60 is arranged to hold a grating G of a PCI system, and a light field projector 61 for projecting a light field towards a light field target area 33. In an embodiment, the holder 60 comprises a first part arranged to hold the light field projector 61 and a second part arranged to hold the grating G. The first part and the second part are permanently or reversibly fixed to each other; either way it provides flexibility in its usage, supply and replacement of parts. The light field projector 61 may be a laser device or a projector or any other type of light field projector. At least one or a plurality of these may be mounted to the holder 60, an example is schematically shown in Fig. 3A. The holder 60 may further comprise a distance sensor 65 (not shown) to measure, when installed in a PCI system, a distance D between the light field projector 61 and the light field target area 33, e.g. part of a patient. In another embodiment, the holder 60 further comprises a guide rail 62 mounted to the holder, configured to allow a movement of the light field projector 61 along the guide rail 62 which comprises a curvature. The laser devices 61 are mounted to the guide rail with a spindle 63 with threads. An exemplary implementation of such spindle 63 with threads along with the guide rail 62 comprising a curvature is illustrated in Fig. 3B. In the exemplary implementation, the spindle with threads is realized through a structure comprising two spindles 63 which are fixedly connected to each other, and a guiding joint 64 to ensure that the spindle position (and hence light field projectors position) as a whole is held. The spindles 63 comprise threads with inverse slopes (S, S') that result in the light field projectors 61 to move in opposite directions (e.g. left/right, top/bottom) when the spindle is turned; the inverse thread slopes S, S' are indicated in the figure by arrows in opposite directions. The structure also comprises fixtures to hold the light field projectors 61 which are mounted to the holder 60 through the guide rail 62. Views from different perspectives of the whole design concept of the guide rail 62 comprising a curvature with a spindle 63 with threads are shown in the figure. In an example, it is through the guide rail 62 with a spindle 63 with threads that the laser devices 61 are mounted to the first part of the holder 60. The purpose of the guide rail 62 comprising a curvature and with a spindle 63 comprising threads with inverse slopes, and the distance sensor 65 will be elucidated when describing Figs. 4 and 5. A person skilled in the art will know that there are alternative implementations to a spindle 63 with threads, for example, one alternative based on a single spindle with two threads of opposing slopes, another based on two non-connected spindles with two independent motors. The motion of the light field projector 61 along the guide rail 62 may be created by other means than a spindle 63 with threads, for instance by linear motors or toothed belts.

Fig. 4A shows a PCI system with optical FOV illumination enabled by a plurality of light field projectors 61. In more detail, in the system of Fig. 4A, an X-ray beam shaper 20, in this case, a collimator, collimates the X-ray beam to a desired FOV through collimator blades, and the light field projector 61 is a laser device 61 configured to project a line shape. The system further comprises the guide rail 62 with a spindle 63 with threads of Fig. 3B mounted to the holder 60, which in this configuration is also the holder of phase grating G1. The guide rail 62 is configured to allow a movement of the laser device 61 along the guide rail 62 and comprises a curvature with a center that coincides with a center of the focal spot O of the X-ray source 10. Four laser devices 61 each projecting a line shape are necessary to cast the FOV border onto the target area 31 of the subject to be imaged. The laser devices 61 are configured to be moved on the guide rail 62 according to the collimator blades position such that the line shapes produced by the laser devices 61 match the edges of the collimator blades. For the four laser devices 61 to move accurately, they are mounted to the first part of the holder through the guide rail 62 with a spindle 63 with threads as that exemplarily shown in Fig. 3B, where the threads have inverse slopes S, S'. More specifically, two of these guide rails 62 are used, with two laser devices 61 mounted on each as shown in Fig. 3B; one of the guide rails 62 mounted at the top or bottom of the holder 60 and the other on the left or right side. By the guide rails 62 sharing the same source point with respect to the X-ray source 10 (i.e. the center of the focal spot O of the X-ray source 10), and through the inverse slopes S, S', a symmetric movement left/right (top/bottom) of the laser line shapes as projected by the laser devices with respect to the optical axis of the system A may be realized. This implementation keeps the mechanical complexity to a minimum. Fig. 4A and 4B show a side and top view perspectives of the system, respectively, where the projected line shapes are shown in dashed lines and are coinciding with the collimated X-ray beam thanks to the described mechanism. The projected line shapes will be seen as perpendicular to the drawing plane and the combined projected light fields will create an area 33 that is correspondent with the radiation area 32, making the visualization of the FOV possible. Therefore, in an embodiment the PCI system comprises a plurality of light field projectors 61 mounted to the holder 60 configured to project a light field on a target area 31 and the combined projected light fields mark an area 33 corresponding to the radiation area 32.

To use the system of Fig. 4 for FOV visualization, a control unit (not shown) may map the blade positions of the collimator 20 to the corresponding laser devices 61 position in the guide rail 62 such that the line shapes produced by the laser devices 61 match the edges of the collimator blades and hence the area 33 that corresponds to the irradiated area 32 is visualized. A calibration procedure may be also performed where for example, the FOV could be illuminated at a plurality of collimator blade openings and for each case, configure the laser devices 61 position to match the edges of the collimator blades. Intermediate positions may be interpolated. As it can be seen from Fig. 3B (e.g. bottom left), the laser devices 61 are mounted such that they are able to move to the same point for the case of the collimator blades being completely closed. Then, the laser devices 61 position may be translated directly into a required number of turns of the spindle 63, making the approach relatively simple to implement. Such a method of use could be performed manually or (semi-)automatically. In an embodiment, the light field projector 61 is configured to update the projected light field 33 when the radiation area 32 is changed (e.g. by moving the collimator blades) to an updated radiation area 32 such that the updated projected light field 33 corresponds to the updated radiation area 32.

The system illustrated in Fig. 4 allows for a very accurate indication of the FOV on the subject with the advantage of this accuracy being independent of the subject position along the optical axis A, since each laser device 61 spans a plane that contains the center of the focal spot O of the X-ray source 10 and the edge of one collimator blade by mechanical motion of the light field projector 61 along the guide rail 62.

An additional advantage of using a laser device as a light field projector 61 is that it offers the option to use the beam when detected with additional sensors at the detector side for alignment and automatic position checks. One check may be without a subject in the laser beam as a regular geometry control, and the second option may be even with the subject in the laser beam as a subject positioning control, for example to see if the subject is in a symmetric position in front of the detector 40. Light intensity that is transmitted at a specific landmark of the subject to be imaged may give further information of the subject characteristics. For example, in the case of the subject being a patient, light intensity that is transmitted at the area of the shoulder/neck can indicate the size/height/position of the patient with respect to the source and patient position. The light intensity may also indicate movements which could guide the patient and/or trigger the acquisition in a moment of no (limited) patient movement.

An alternative to FOV visualization without relying on the mechanical motion of the laser device 61 along the guide rail 62 is illustrated in the PCI system of Fig. 1A. In the figure, the holder 60 is arranged to hold the light field projector 61 and phase grating G1, and a collimator 20, collimates the X-ray beam to a desired FOV through the collimator blades. To avoid relying on the mechanical motion of the light field projector 61, the system further comprises measuring a distance D from a plane B where the light field is projected to a plane C where the target area 31 is located and calculating a projection angle of the light field projector 61 (e.g. laser device 61) such that the projected light field 33 corresponds to the area to be irradiated 32; in other words, the FOV setting, i.e. the collimator blades position of the collimator 20 is mapped to a projection angle of the light field projector 61 (e.g. laser device 61) for a particular distance D. To measure said distance D, the system of Fig. 1A further comprises a distance sensor 65. In another example (not shown), at least one laser device 61 mounted to the first part of the holder 60 is configured to project a collimated laser spot towards the light field target area 33, and is also configured to scan over the FOV such that a border of the FOV is visualized. The laser spot should scan quickly enough such that to the eye of an observer it appears as a solid line. Similarly, the distance D from the plane B where the light field is projected to the plane C where the target area 31 is located needs to be measured and the scanning range (e.g. starting and final scanning x-y positions) determined such that the projected light field 33 corresponds to the area to be irradiated 32. In an embodiment, the PCI system further comprises a distance sensor 65 to measure the distance from a plane B where the light field is projected to a plane C where the target area 31 is located. Clearly, the distance D measurement is needed in the cases where the projected light field does not have the same source point as the X-ray source 10 (i.e. the center of the focal spot O of the X-ray source 10); considering this distance when projecting a light field with a source point different from that of the X-ray source, will contribute to the correct FOV visualization.

Fig. 5A illustrates a PCI system with optical FOV visualization enabled by a light field projector 61 comprising a projector 61 which is held by the holder 60 of phase grating G1. Like in the system of Fig. 4, a collimator 20, collimates the X-ray beam to a desired FOV through the collimator blades. Then, the set FOV is mapped to an image to be projected by the projector 61 thereby forming a light field covering and delimiting the complete FOV 33 that is correspondent with the radiation area 32. A change in the FOV 32 will be mapped to a different image to be projected 33 onto the subject and hence there is no need for mechanical motion of the optical elements. Yet, the image projected by the projector does not have the same source point as the X-ray source. Thus, the FOV may be visualized correctly only in a position (i.e. a specific distance D from the plane B where the light field is projected to the plane C where the target area is located) of the subject along the optical axis A. Exemplarily, in the arrangement as sketched in Fig. 5A, the projected area has an error larger at the bottom than at the top. A more detailed analysis using realistic assumptions about the geometry shows that an accuracy of ±1 cm can be achieved at the bottom if the subject position along the optical axis A does not deviate from the desired position by more than ±5 cm. On the other hand, the upper end is projected with an accuracy of ±1 cm as long as the subject does not deviate from the desired position by more than ±22 cm. Of course, these tolerances change depending on the collimation (i.e. FOV); in particular, the ranges will shrink as the collimation is narrowed.

Fig. 5B shows a sketch of a more accurate implementation comprising a plurality of projectors 61; two projectors 61 project independently (partially overlapping) parts of the FOV onto the subject. The combination of the independently projected light fields covers and delimits an area 33 as a whole, where the projected borders of the FOV lie in the regions with less parallax, hence, the overall accuracy is improved.

In yet another embodiment (not shown), the system comprises four projectors 61 mounted to the holder 60 (e.g. arranged as in Fig. 3A) to achieve a high accuracy for all four borders of the FOV. Since the distance D from the plane B where the light field is projected to the plane C where the target area is located further contributes to an accurate FOV visualization, a more advanced embodiment comprises a distance sensor 65 as sketched in Fig. 5B.

The use of a projector 61 does not only allow the visualization of the FOV but it may also allow the projection of additional information on or near the light field target area 33. For example, additional information can include the alignment to the patient's body with projection of the body shape; based on complementary subject data, the projection of a target region (e.g. a body part) to favor the application of the correct FOV setting; or any other relevant data for the confirmation of the examination (e.g. a patient's name, purpose of scan, date of birth, etc.). Further, the projector 61 could be complemented with a feedback device (e.g. a camera) that allows an operator to control the FOV setting via gesture control at the back of the subject (e.g. like action on a touch screen). Besides the increased functionality by projection of additional information, such a user interface improves the workflow and allows for a faster and more precise FOV setting which could be fully automated.

To use an embodiment of the system for FOV visualization that does not rely on the mechanical motion of the light field projectors, such as that described in Figs. 1A and 5, a control unit (not shown) may map the FOV setting to the FOV illumination setting for a specific distance D between the light field projector 61 and the target area 31, such that the projected area 33 corresponds to the radiation area 32. For example, the control unit may map a FOV setting such as collimator blade positions to a FOV illumination setting such as an image to be projected by a projector 61, or to a projection angle of a laser device 61, or to a scanning range of a laser device 61, etc. A measurement from a distance sensor 65 could be also monitored by the control unit for a more accurate implementation. A calibration procedure or a lookup table could be implemented for the purpose of mapping the FOV setting with the FOV illumination setting. Further, the FOV illumination setting can be configured to be updated when the radiation area 32 is changed to an updated radiation area 32 such that the updated illuminated area 33 corresponds to the updated radiation area 32. The method of use could be performed manually or (semi-) automatically.

It is noted that the imaged 'subject' in the context of the invention may refer to a dead or living human or animal body/patient or a part thereof, or to a plant or portion of a plant. Furthermore, it may stand for an inanimate subject such as an item or luggage in a security screening system or a sample object in non-destructive material testing. Therefore, although the claimed invention is directed towards medical applications, it is also very suitable for application in non-destructive testing (e.g. analysis as to composition, structure and/or qualities of biological and well as non-biological samples) as well as security scanning (e.g. scanning of luggage on airports).

It has to be noted that embodiments of the invention are described with reference to different subject matters. In particular, some embodiments are described with reference to system type claims whereas other embodiments are described with reference to the device type claims. However, a person skilled in the art will gather from the above description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or
exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A phase contrast X-ray imaging system comprising:
- an X-ray source (10) configured to emit an X-ray beam through an examination region (50) towards a target area (31) located within the examination region;
- an X-ray detector (40) positioned on an opposite side of the examination region from the X-ray source;
- an X-ray beam shaper (20) configured to shape the X-ray beam to irradiate a radiation area (32) on the target area;
- a grating (G) positioned between the X-ray source and the detector;
- a light field projector (61), configured to project a light field in the visible spectrum on the target area, wherein the projected light field corresponds to the radiation area;
- a holder (60), arranged to hold the light field projector and the grating.

2. The system of claim 1, wherein the holder (60) contains a first part arranged to hold the light field projector (61) and a second part arranged to hold the grating (G), wherein the first part and the second part are permanently or reversibly fixed to each other.

3. The system of any of the previous claims, wherein the grating (G) is a phase grating (G1) or a source grating (G0) or a sample absorbing grating (SG).

4. The system of any of the previous claims, wherein a plurality of light field projectors (61) is mounted to the holder (60) configured to project a light field on a target area (31), and wherein the combined projected light fields mark an area corresponding to the radiation area (32).

5. The system of any of the previous claims, further comprising a distance sensor (65) to measure a distance from a plane (B) where the light field is projected to a plane (C) where the target area (31) is located.

6. The system of any of the previous claims, wherein the light field projector (61) is a laser device or a projector.

7. The system of any of the previous claims, wherein the light field projector (61) is a laser device (61) configured to project a line shape and the system further comprises a guide rail (62) mounted to the holder (60), wherein said guide rail is configured to allow a movement of the laser device along the guide rail which comprises a curvature with a center that coincides with a center of a focal spot (O) of the X-ray source.

8. The system of any of the previous claims wherein the light field projector (61) is configured to update the projected light field when the radiation area (32) is changed to an updated radiation area such that the updated projected light field corresponds to the updated radiation area.

9. A holder (60) arranged to hold:
- a grating (G) of a phase contrast X-ray imaging system; and
- a light field projector (61) for projecting a light field towards a light field target area (33).

10. The holder of claim 9, wherein the holder (60) contains a first part arranged to hold the light field projector (61) and a second part to hold the grating (G), wherein the first part and the second part are permanently or reversibly fixed to each other.

11. The holder of claims 9 and 10, wherein the light field projector (61) is a laser device or a projector.

12. The holder of claims 9 to 11, further comprising a guide rail (62) mounted to the holder (60), configured to allow a movement of the light field projector (61) along the guide rail which comprises a curvature.

13. The holder of claim 12, wherein the light field projector (61) comprises a plurality of laser devices (61) configured to project a line shape, that are mounted to the first part through the guide rail (62) with a spindle (63) with threads, wherein the threads have inverse slopes (S, S') to allow, when installed in a phase contrast X-ray imaging system, a symmetric movement of the projected laser line shapes with respect to an axis coincident with an optical axis (A) of the phase contrast X-ray imaging system.

14. The holder of claims 9 to 13 further comprising a distance sensor (65) to measure, when installed in a phase contrast X-ray imaging system, a distance (D) between the light field projector (61) and the light field target area (33).

15. The holder of claims 11-14 wherein the light field projector (61) comprises at least one laser device (61) mounted to the first part of the holder 60, configured to project a collimated laser spot towards the light field target area (33), and to scan over the light field target area such that a border of the light field target area is visualized.

16. The holder of claims 11-14 wherein the light field projector (61) comprises at least one projector (61) mounted to the first part of the holder 60, configured to project a light field towards the light field target area (33) to form a light field covering and delimiting the complete light field target area.

17. The holder of claim 16, wherein at least one of the at least one projector (61) projects further information on or near the light field target area (33), such as projection of a target region, alignment to a subject's body with projection of a body shape, and/or other data.
